# EUROPÄISCHE PATENTANMELDUNG

(11) **EP 0 786 258 A1**
(43) Veröffentlichungstag der Anmeldung: **30.07.1997**
(21) Anmeldenummer: 97100457.7
(22) Anmeldetag: 14.01.1997
(51) Int. Cl.: A61L 2/18, G02C 13/00

(54) **Verfahren zur Pflege einer Anpasskontaktlinse und Pflegesystem hierfür**

(30) Priorität: 17.01.1996 DE 19601568
(71) Anmelder: Müller-Lierheim, Wolfgang G.K., Dr., 81477 München (DE)
(72) Erfinder: Müller-Lierheim, Wolfgang G.K., Dr., 81477 München (DE)
(74) Vertreter: Nöth, Heinz, Dipl.-Phys.

(57) **Zusammenfassung**

Ein Verfahren und ein System zur Pflege einer Anpaßkontaktlinse, bei dem die Linse mit einer Wasserstoffperoxidlösung (insbesondere 3 %) desinfiziert wird, besitzt das Pflegesystem neben einem Aufbewahrungsbehälter 1 einen Desinfektionsbehälter 2, in welchem mit der H₂O₂-Lösung die Anpaßkontaktlinse desinfiziert wird, sowie einen mit einem Linsenhalter 3 versehenen Behälterverschluß 4, mit welchem die desinfizierte Anpaßkontaktlinse während der Desinfizierung im Desinfektionsbehälter 2 gehalten wird und von dort zur Neutralisierung von an der desinfizierten Anpaßkontaktlinse verbliebenen H₂O₂-Resten in den Anpaßbehälter 1 gebracht werden kann, in welchem ein zur Aufspaltung der H₂O₂ dienender Neutralisationskatalysator, beispielsweise in Form einer Platinbeschichtung 5, vorgesehen ist.

## Beschreibung

Die Erfindung betrifft ein Verfahren nach dem Oberbegriff des Patentanspruches 1 und ein Pflegesystem nach dem Oberbegriff des Patentanspruches 3.

Bei der Kontaktlinsenanpassung durch einen Optiker oder Augenarzt werden für die Pflege der Anpaßkontaktlinse folgende Hygienemaßnahmen getroffen: Die Anpaßlinse wird aus der Aufbewahrungslösung herausgenommen und mit Reinigungslösung, die mit dem Kontaktlinsenpflegemittel oder zum Teil mit der Aufbewahrungslösung identisch sein kann, abgespült. Die Anpaßlinse wird dann auf den Augenbulbus aufgesetzt und etwa eine Stunde zur Probe getragen. Danach wird die Linse entnommen und mit den Fingern unter Reibebewegungen gereinigt. Anschließend wird erneut mit Reinigungslösung abgespült, und die Linse wird dann in den Behälter des Anpaßsatzes mit erneuerter Aufbewahrungslösung bis zum nächsten Anpaßvorgang gegeben. Eine Reinigung (Satzreinigung) des kompletten Anpaßsatzes - unabhängig davon, ob die Linsen in Gebrauch waren oder nicht - erfolgt in regelmäßigen im allgemeinen mehrwöchigen Abständen. A. Kramer et al berichten in einem Aufsatz 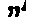 Anpassung weicher Kontaktlinsen - ein bisher vernachlässigtes Infektionsrisiko" (abgedruckt in Hyg Med 20. Jahrgang 1995, Heft 6, Seiten 278 bis 291) über Ergebnisse mikrobiologischer Analysen von Aufbewahrungslösungen von Anpaßkontaktlinsen bei Optikern und Augenärzten. Aufgrund der ungenügenden mikrobiziden Wirksamkeit der Abspül- bzw. Aufbewahrungslösungen und des mangelhaften Pflegesystems ergaben sich hohe Kontaminationsraten mit Bakterien und Pilzen. Als Kontaminationsquellen kommen sowohl der behandelnde Optiker bzw. Augenarzt als auch der Patient in Frage. Als geeignetes Pflegesystem zum Sterilisieren bzw. Desinfizieren der Linsen bietet sich das Autoklavieren der Linsen an. Dieses Verfahren ist jedoch nicht für alle Linsen geeignet. Dies gilt insbesondere für harte und gasdurchlässige Linsen. Auch Weichlinsen altern durch mehrmaliges Autoklavieren schneller als bei chemischer Desinfektion. Außerdem sind hierfür relativ aufwendige Autoklaven erforderlich, die nicht überall verfügbar oder aufstellbar sind.

Von den chemischen Desinfektionsmitteln bieten sich derzeit verfügbare Desinfektionslösungen auf H₂O₂-Basis (3-%ige Lösungen), wie sie bei Kontaktlinsen zum Einsatz kommen, an. Derartige Desinfektionslösungen werden bei der Kontaktlinsenpflege anschließend oder gleichzeitig neutralisiert. Die Neutralisation erfolgt katalytisch (z.B. Platinkatalysator) oder enzymatisch (z. B. Katalase) durch Aufspaltung in Wasser und Sauerstoff. Bei Systemen mit gleichzeitiger Neutralisation befindet sich die Kontaktlinse nach der Neutralisation in unkonservierter Lösung. Reste nicht abgetöteter Keime und/oder Protozoen aus dem Kopfraum des Kontaktlinsenbehälters, die keinen Kontakt zum H₂O₂ hatten, können sich während der Lagerung der Kontaktlinse in der neutralisierten Lösung vermehren und ein Kontaminationsrisiko darstellen. Der Zusatz von Konservierungsmitteln zum Wasserstoffperoxid scheitert an der Oxidationswirkung des H₂O₂, die zu einer Zerstörung des Konservierungsmittels führt. Die längerfristige Lagerung der Linse in H₂O₂ hat den Nachteil, daß das Wasserperoxid den Kontaktlinsenkunststoff vorzeitig altert und daß die Linse nach der Entnahme aus dem Anpaßsatz erst neutralisiert werden muß, bevor sie einem Träger ins Auge gesetzt werden kann.

Aufgabe der Erfindung ist es, ein Verfahren und ein Pflegesystem der eingangs genannten Art zu schaffen, bei welchem mit geringem Aufwand und einfacher Handhabung das Infektionsrisiko beim Anpaßvorgang erheblich verringert wird.

Diese Aufgabe wird erfindungsgemäß beim Verfahren durch die kennzeichnenden Merkmale des Patentanspruches 1 und beim Pflegesystem durch die kennzeichnenden Merkmale des Patentanspruches 3 gelöst.

Durch die Erfindung wird eine einfache rasche und wirksame Desinfizierung bzw. Sterilisierung der Anpaßkontaktlinse erreicht, wobei der erreichte Hygienestatus sich über einen längeren Zeitraum aufrecht erhalten läßt. Das Pflegeverfahren und Pflegesystem sind für alle bislang bekannten Anpaßkontaktlinsen geeignet und gewährleisten die erforderliche Sicherheit des Kontaktlinsenträgers vor Krankheitsübertragung bei der Linsenanpassung.

Das Pflegesystem besteht aus der Desinfektionslösung auf Wasserperoxid-Basis. Diese Desinfektionslösung enthält H₂O₂ (vorzugsweise 3 %) in wäßriger, gepufferter, isotonischer Lösung mit vorzugsweise saurem bis neutralem pH-Wert. Als zweite Lösung enthält das Pflegesystem eine konservierte Lösung, bei der es sich bevorzugt um eine konservierte, gepufferte, isotonische Kochsalzlösung mit annähernd neutralem pH-Wert handelt.

Ein weiterer Bestandteil des Pflegesystems ist ein Linsenbehälter, der die Funktion eines Desinfektionsbehälters hat, in welchem die Desinfektion der Anpaßkontaktlinse nach dem Anpaßvorgang in der Wasserstoffperoxid enthaltenden Desinfektionslösung durchgeführt wird. Zum Pflegesystem gehört ein weiterer Linsenbehälter, welcher die Funktion des Aufbewahrungsbehälters für die Anpaßkontaktlinse im Anpaßlinsensatz hat. In diesem Aufbewahrungsbehälter erfolgt auch die Neutralisation von H₂O₂-Resten an der Anpaßkontaktlinse, nachdem diese aus dem Desinfektionsbehälter entnommen und in den mit der konservierten Lösung gefüllten Anpaßbehälter gebracht worden ist.

Ferner gehört zum Pflegesystem ein Linsenhalter, mit welchem die Linse beim Eintauchen in die Wasserstoffperoxidlösung im Desinfektionsbehälter gehalten wird und mit welchem unter Vermeidung der Berührung der Linse diese aus dem Desinfektionsbehälter in den Aufbewahrungsbehälter gebracht werden kann. In bevorzugter Weise ist der Linsenhalter mit einem Behälterverschluß verbunden, mit welchem sowohl der Desinfektionsbehälter als auch der Aufbewahrungsbehälter verschlossen werden können. In bevorzugter Weise ist der Behälterverschluß als Schraubverschluß (Schraubdeckel) ausgebildet.

An der Oberseite des Behälterverschlusses sind Vorrichtungen für ein Beschriftungssystem vorhanden. In diesem Beschriftungssystem können die Parameter der Linse, die Historie der Anpaßvorgänge mit der Linse, das Datum der letzten Desinfektionsbehandlung und die Identität der Person, welche die Desinfektion durchgeführt hat, angegeben werden.

In üblicher Weise können eine Standardarbeitsanweisung und ein Schulungspaket über Hygienemaßnahmen bei der Kontaktlinsenanpassung vorgesehen sein.

Der Ablauf der Pflege der Anpaßkontaktlinse erfolgt bevorzugt in der folgenden Weise:

Der Anpasser nimmt die Anpaßkontaktlinse aus dem Aufbewahrungsbehälter und spült sie vor dem Einsetzen bevorzugt mit der konservierten Lösung ab. Nach dem Anpaßvorgang wird die Linse mit einem für die Linse geeigneten Reiniger gereinigt und mit der konservierten Lösung abgespült. Anschließend wird die Linse in den mit dem Behälterverschluß verbundenen Linsenhalter eingebracht und in eine im Desinfektionsbehälter eingefüllte Desinfektionslösung (3-%ige H₂O₂-Lösung) eingetaucht. Der Deckelverschluß wird dabei beispielsweise durch Aufschrauben auf den Desinfektionsbehälter aufgesetzt, wobei gleichzeitig die im Linsenhalter befindliche Anpaßkontaktlinse in die Desinfektionslösung eingetaucht wird. Am Beschriftungssystem des Behälterverschlusses können die entsprechenden Daten eingegeben werden.

Nach Abschluß der Desinfektionsbehandlung, die vorzugsweise über Nacht geschieht, wird die Anpaßkontaktlinse durch Abnehmen des Deckelverschlusses vom Desinfektionsbehälter aus diesen entnommen und in den Aufbewahrungsbehälter gebracht, in welchem die konservierte Lösung eingefüllt worden ist.

Der Aufbewahrungsbehälter enthält ein Neutralisationsmittel, beispielsweise in Form eines Katalysators, z.B. Platin-Katalysator, mit welchem innerhalb kurzer Zeit Reste von Wasserstoffperoxid, die an der Linse haften geblieben sind, durch Aufspaltung in Wasser und Sauerstoff neutralisiert werden. In der konservierten Lösung des Aufbewahrungsbehälters kann die Anpaßkontaktlinse bedenkenlos bis zu drei Monaten aufbewahrt werden, ohne daß eine erneute Desinfektion erforderlich ist.

Die beiden Behälter (Desinfektionsbehälter, Aufbewahrungsbehälter) und der Behälterverschluß sind bevorzugt aus autoklavierbarem Material. Der Behälterverschluß kann aus Kunststoff, z.B. Polycarbonat, bestehen.

Anhand der Figuren wird an einem Ausführungsbeispiel die Erfindung noch näher erläutert. Es zeigt:
- Fig. 1:: die beiden zum Pflegesystem gehörigen Behälter mit einem wechselweise auf die Behälter aufsetzbaren Behälterverschluß;
- Fig. 2:: die Unterseite des Behälterverschlusses;
- Fig. 3:: den Behälterverschluß mit geschlossenem Linsenhalter; und
- Fig. 4:: den Behälterverschluß mit geöffnetem Linsenhalter.

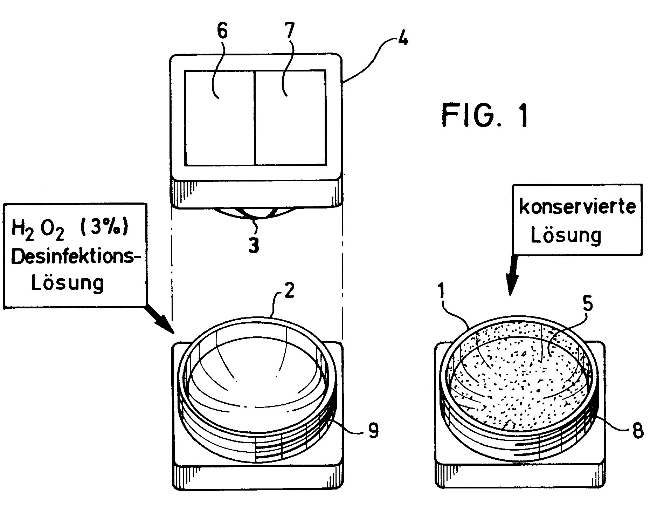

In der Fig. 1 sind die beiden Linsenbehälter, nämlich ein Aufbewahrungsbehälter 1 und ein Desinfektionsbehälter 2, welche zum Pflegesystem gehören, als Ausführungsbeispiele dargestellt. Der Aufbewahrungsbehälter 1 ist an seiner Innenseite mit einer Platinschicht 5 versehen. Diese Platinschicht ist bevorzugt durch Sputtern aufgebracht. Die Platinschicht 5 wirkt als Neutralisationsmittel in Form eines Katalysators, der die Aufspaltung von H₂O₂ in Wasser und Sauerstoff bewirkt. Der Katalysator kann auch in Form eines beschichteten, insbesondere durch Sputtern beschichteten Kunststoffkörpers (DE 195 22 950) vorhanden sein. Im Aufbewahrungsbehälter wird die Anpaßkontaktlinse zwischen den einzelnen Anpaßvorgängen aufbewahrt. Ferner wird im Anpaßbehälter 1 aufgrund der katalytischen Wirkung der Platinbeschichtung 5 die Neutralisierung von H₂O₂-Resten, welche nach der Desinfektionsbehandlung an der Anpaßlinse haften durchgeführt. Sowohl bei der Neutralisationsbehandlung als auch bei der Aufbewahrung der Anpaßlinse befindet sich die Linse in einer konservierten Lösung, die insbesondere als konservierte, gepufferte, isotonische Kochsalzlösung mit annähernd neutralem pH-Wert ausgebildet ist, im Aufbewahrungsbehälter 1. Die Anpaßkontaktlinsen des Anpaßsatzes sind bevorzugt in derartigen Aufbewahrungsbehältern 1 angeordnet.

Ein zweiter zum Pflegesystem gehöriger Behälter, welcher die Funktion eines Desinfektionsbehälters 2 hat, ist ebenfalls Bestandteil des Pflegesystems. Für die Desinfektionsbehandlung der Anpaßkontaktlinse wird in den Desinfektionsbehälter 2 eine vorzugsweise 3 %ige Wasserstoffperoxidlösung verwendet.

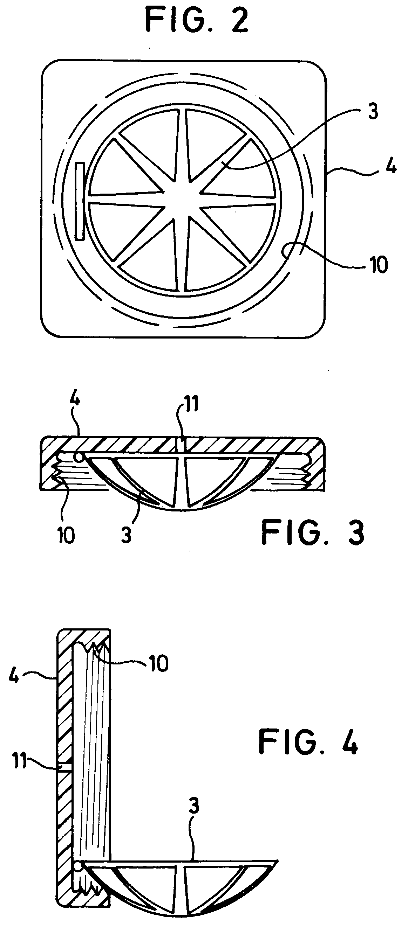

Um die Anpaßkontaktlinse zur jeweiligen Behandlung in den Anpaßbehälter 1 und in den Desinfektionsbehälter 2 wechselweise ohne Gefahr der manuellen Berührung der Anpaßkontaktlinse einbringen zu können, ist ein Behälterverschluß 4 vorgesehen, an welchem in Form eines Körbchens ein lösbarer Linsenhalter 3 vorgesehen ist (Figuren 2 bis 4). Der als Schraubdeckel ausgebildete Behälterverschluß 4 besteht bevorzugt aus autoklavierbarem Kunststoff, z.B. Polycarbonat. Der Behälterverschluß 4 besitzt in Form einer kleinen Bohrung 11 eine Öffnung, um insbesondere bei der Desinfektionsbehandlung das Entstehen eines Überdrucks im Behälter zu vermeiden. Am Behälterverschluß ist in Form eines Körbchens der Linsenhalter 3 schwenkbar befestigt. Bei der in der Fig. 4 dargestellten geöffneten Position des Linsenhalters 3 kann die Linse in den Linsenhalter eingelegt werden. In der in der Fig. 3 dargestellten geschlossenen Position des Linsenträgers 3 wird der Behälterverschluß 4 mit dem an der Unterseite befindlichen Linsenhalter 3 zur jeweiligen Behandlung auf einen der beiden Behälter 1 und 2 aufgesetzt. Der Aufbewahrungsbehälter 1 besitzt an seinem oberen Rand ein Außengewinde 8, und der Desinfektionsbehälter 2 besitzt an seinem oberen Rand ein Außengewinde 9. Der Behälterverschluß 4 besitzt ein Innengewinde 10. Durch Aufschrauben kann der Behälterverschluß 4 wechselweise auf die beiden Behälter 1 und 2 aufgeschraubt werden. Beim Aufschrauben wird die im Linsenträger 3 befindliche Anpaßkontaktlinse in die jeweilige Lösung, welche sich im Behälter 1 bzw. im Behälter 2 befindet, eingetaucht. Der Linsenhalter 3 ist hierzu durchlässig für die konservierte Lösung im Aufbewahrungsbehälter 1 und für die Desinfektionslösung im Desinfektionsbehälter 2. Beim dargestellten Ausführungsbeispiel besteht der korbförmige Linsenträger aus einem sternförmigen kalottenartig geformten Mittelteil, der von einem Ring zusammengehalten wird. Durch Aufklappen, wie in der Fig. 4 oder durch einen Schnappverschluß kann der Linsenträger 3 lösbar mit dem Behälterverschluß 4, welcher als Schraubdeckel ausgebildet ist, verbunden werden.

An der Außenseite des quadratisch mit gerundeten Ecken ausgebildeten Deckels des Behälterverschlusses befindet sich ein Dokumentations- und Beschriftungssystem, bestehend aus zwei rechteckigen, bevorzugt farbig ausgebildeten Scheibchen 6 und 7 aus Karton oder Kunststoff, die wiederverwendbar am Behälterverschluß 4 vorgesehen sind. Eines der beiden Scheibchen, beispielsweise das Scheibchen 6, enthält die für die Anpaßlinse wichtigen Parameter, insbesondere die Basiskurve, den Durchmesser und die Stärke (dpt) der Anpaßkontaktlinse und gegebenenfalls Hinweise auf das Linsenmaterial. Am zweiten Beschriftungsscheibchen können Daten zur Historie der Anpaßkontaktlinse, wie z.B. Anzahl der Verwendungen, Daten der letzten Desinfektion und Angaben zur Identität der Person, die die letzte Desinfektion durchgeführt hat, vorgesehen sein. Die Beschriftungsscheibchen 6 und 7 können unter zwei herausnehmbare optisch klare rechteckige Scheibchen zur Befestigung am Deckel des Behälterverschlusses 4 eingelegt werden.

Es kann auch ein zusätzliches Dokumentationssystem zur Verfügung gestellt sein, mit welchem sich die Geschichte jeder Anpaßkontaktlinse zurückverfolgen läßt.

Da auch mehrere Anpaßlinsen zur Anpassung in die Augen des Kontaktlinsenträgers im Verlauf eines Anpaßvorganges nacheinander eingessetzt werden müssen, können in bevorzugter Weise mehrere Desinfektionsbehälter 2 vorgesehen sein, so daß nach jeweiligen Anpaßvorgängen gleichzeitig mehrere Anpaßkontaktlinsen desinfiziert bzw. sterilisiert werden können.

In bevorzugter Weise besitzt jeder Aufbewahrungsbehälter 1 des Anpaßlinsensatzes einen Behälterverschluß 4 mit zugeordnetem Linsenhalter 3.

## Patentansprüche

1. Verfahren zur Pflege einer Anpaßkontaktlinse, bei dem mit einer H₂O₂-Lösung die Linse desinfiziert und anschließend H₂O₂-Lösungsmittelreste an der Linse neutralisiert werden,
dadurch **gekennzeichnet,** daß
nach dem Anpaßvorgang die jeweilige Linse in einen kennzeichenbaren Linsenhalter, welcher für die H₂O₂-Lösung durchlässig ist, eingebracht und in die H₂O₂-Lösung zur Desinfektionsbehandlung eingetaucht wird, und daß die Linse im Linsenhalter verbleibend aus der H₂O₂-Lösung entfernt und in eine konservierte Lösung getaucht wird, in welcher gleichzeitig an der Linse vorhandene H₂O₂-Lösungsreste neutralisiert werden.

2. Verfahren nach Anspruch 1, dadurch gekennzeichnet, daß die Anpaßkontaktlinse in dem Behälter mit der konservierten Lösung bis zu einem nachfolgenden Anpaßvorgang aufbewahrt wird.

3. Pflegesystem für eine Anpaßkontaktlinse mit wenigstens einem Aufbewahrungsbehälter für die Linse, einer H₂O₂-Lösung zur Desinfektion der Linse und einem Neutralisationsmittel zur Neutralisation von H₂O₂,
dadurch **gekennzeichnet,** daß
zusätzlich zum Aufbewahrungsbehälter (1) ein Desinfektionsbehälter (2), in welchem die H₂O₂-Lösung einfüllbar ist, vorgesehen ist, daß ein Linsenträger (3) an einem kennzeichenbaren Behälterverschluß (4) befestigt ist, mit welchem beide Behälter (1, 2) nacheinander verschließbar sind, und daß in dem Aufbewahrungsbehälter (1), der ein H₂O₂ neutralisierendes Mittel enthält, eine konservierte Lösung eingefüllt ist.

4. Pflegesystem nach Anspruch 3, dadurch gekennzeichnet, daß der Linsenträger (3) am Behälterverschluß (4) in der Weise befestigt ist, daß beim Wechseln des Behälterverschlusses zwischen den beiden Behältern (1, 2) eine Berührung der vom Linsenträger (3) gehaltenen Linse vermeidbar ist.

5. Pflegesystem nach Anspruch 3 oder 4, dadurch gekennzeichnet, daß am Behälterverschluß (4) auswechselbare Etiketten befestigbar sind.

6. Pflegesystem nach einem der Ansprüche 3 bis 5, dadurch gekennzeichnet, daß der Behälterverschluß (4) als Schraubverschluß ausgebildet ist.

7. Pflegesystem nach einem der Ansprüche 3 bis 6, dadurch gekennzeichnet, daß der Aufbewahrungsbehälter (1) einen H₂O₂ neutralisierenden Katalysator aufweist.

8. Pflegesystem nach Anspruch 7, dadurch gekennzeichnet, daß der Katalysator als Schicht an der Behälterwand vorgesehen ist.
